# EUROPEAN PATENT SPECIFICATION

(11) **EP 2 906 183 B1**
(45) Date of publication and mention of the grant of the patent: **20.03.2019**
(21) Application number: 13802253.8
(22) Date of filing: 10.10.2013
(51) Int. Cl.: A61K 8/36, A61Q 15/00, A61K 8/97

(54) **COSMETIC USE OF A MONOUNSATURATED FATTY ACID OR ONE OF ITS SALTS AND/OR OF ITS ESTERS OR AMIDES AS DEODORANT ACTIVE AGENT**
KOSMETISCHE VERWENDUNG EINER EINFACH GESÄTTIGTEN FETTSÄURE ODER EINES IHRER SALZE UND/ODER ESTER ODER AMIDE ALS DESODORIERENDER WIRKSTOFF
UTILISATION COSMÉTIQUE D'UN ACIDE GRAS MONOINSATURÉ OU D'UN DE SES SELS ET/OU DE SES ESTERS OU AMIDES EN TANT QU'AGENT ACTIF DÉODORANT

(30) Priority: 15.10.2012 FR 1259796; 17.10.2012 US 201261715025 P
(43) Date of publication of application: 19.08.2015
(73) Proprietor: L'Oréal, 75008 Paris (FR)
(72) Inventor: GUENICHE, Audrey, F-92500 Rueil Malmaison (FR); CASTIEL, Isabelle, F-06200 Nice (FR)
(74) Representative: Brohmi, Karim
(86) International application number: PCT/EP2013/071125
(87) International publication number: WO 2014/060276

(56) References cited:
- EP-A2- 1 428 521
- WO-A1-02/102337
- WO-A1-2011/067105
- GB-A- 2 262 887
- US-A- 5 958 386
- DATABASE GNPD [Online] MINTEL; 31 December 2011 (2011-12-31), "Anti-perspirant Deodorant", XP002707732, Database accession no. 1684243

## Description

A subject-matter of the present invention is the cosmetic use, as deodorant active agent, of a monounsaturated fatty acid or one of its salts and/or of its esters and/or its amides, in particular in a composition comprising a physiologically acceptable medium.

It also relates to a cosmetic method by the oral route for treating and/or preventing unpleasant human body odours, consisting in ingesting an oral composition comprising, in a physiologically acceptable medium, at least one monounsaturated fatty acid or one of its salts and/or of its esters and/or its amides.

It also relates to a cosmetic method by the topical route for treating and/or preventing unpleasant human body odours, consisting in applying, to the surface of a human keratinous substance, a composition comprising, in a physiologically acceptable medium, at least one monounsaturated fatty acid or one of its salts and/or of its esters.

Perspiration (or sweating) is the elimination of sweat via the pores of the skin. This physiological function is present at various degrees in all mammals. In man, the main function is temperature regulation and the elimination of toxins. Sweat is a liquid secreted by two types of sweat glands. The eccrine glands are located over the entire body surface, eccrine sweat is transparent, aqueous and odourless and plays a role in the hydration of the horny layer and in microbiological protection, in grasping and in percutaneous absorption. The apocrine glands are more particularly located in the armpits, on the auditory canals, in the mammary areolae and in the perianal regions. The excretory part is located at the upper level of the hair follicle. These glands only become functional after puberty. Apocrine sweat is viscous, milky in appearance and not very odorous. It is rich in lipids and also comprises proteins. This secretion is psychologically regulated. Sweat is sterile and is not or not very odorous during its secretion. Body odours are due to the bacterial decomposition of apocrine sweat. Humidity, sebaceous secretions, hairiness, hygiene and diet are also responsible for body odours.

The axillary region is the point of the body where the most bacteria are found. The latter produce volatile and malodorous compounds.

The axillary regions comprise a wide range of bacteria (approximately 10⁶ cells/cm²), the majority of which are Gram+ bacteria. These bacteria comprise the following groups: *Staphylococcus* species, *Micrococcus* species, anaerobic/microaerobic *Propionibacterium* species; and aerobic coryneform bacteria which are themselves composed of *Corynebacterium* species (83%), *Brevibacterium* species (5%) and other coryneforms (12%). The axillary flora also comprises the yeast of the genus *Malassezia.*

Studies have shown that the majority of axillary coryneforms are comparable either to *Corynebacterium mucifaciens* or *Corynebacterium striatum* and *Corynebacterium jeikeium.*

A correlation exists between the number of corynebacteria and the intensity of the odours formed. This correlation does not exist for other microorganisms. The pH 6 is the optimum pH when the production of odours by coryneforms takes place.

People having an unpleasant axillary odour have significantly more microorganisms than those not having it. In addition, the composition of the flora of these two populations is different. The percentage of lipophilic diphtheroids is much higher in people having an unpleasant odour but *Staphylococcus epidermidis* are also found in high number.

The compounds which contribute to unpleasant axillary odours comprise the molecules such as volatile short-chain C₂-C₅ fatty acids, malodorous steroids (16-androstene steroids), C₆-C₁₂ acids, including the trans (E) isomer of 3-methyl-2-hexenoic acid (3M2H), or sulfanylalkanols.

Body odours are biological phenomena which it appears important to effectively control. In order to combat perspiration and unpleasant odours, various compounds have already been provided which, by topical application to the skin, are capable of inhibiting sweating, of inhibiting the bacteria responsible for the chemical transformations, of masking the odours or of reducing/destroying the indigenous microflora.

To affect unpleasant odours, provision has been made, generally:
- to affect the sweat glands by blocking the perspiration process or at least by regulating it
- to affect the decomposition of the sweat by using enzymatic or non-enzymatic bactericides
- to affect odour emission: fragrances or odour absorbers.

The most commonly employed bactericide is triclosan (2,4,4-trichloro-2-hydroxydiphenyl ether), which has the disadvantage of modifying the entire cutaneous microflora and of being inhibited by certain compounds often used in cosmetics.

It is also possible to block volatile fatty acids by adding, at the perspiration areas, compounds capable of trapping volatile fatty acids or else of raising the pH in order to prevent the formation of volatile fatty acids and to prevent the proliferation of the bacterial flora.

In order to affect the sweat by reducing or regulating perspiration, use may be made of active substances of antiperspirant type which have the effect of limiting the flow produced. They generally have, as active principles, aluminium and/or zirconium salts or complexes, which reduce the flow of sweat by modifying the cutaneous physiology. The other denaturing substances used include glutaraldehyde and formaldehyde.

There exist other treatments, such as sympathectomy and excision, curettage or liposuction of the areas most active in the secretion of sweat. There exists, however, a risk of onset of compensatory phenomena, such as the formation of cheloids, a pneumothorax or hyperhidrosis or compensatory hyperhidrosis.

Finally, it is possible to affect the phenomenon of sweating by use of toxin, of tranquillisers, of sedatives/spasmolytics or of anticholinergics, which often result in a great many side-effects.

A need thus remains to have available novel active agents capable of exerting a cosmetic action in order to affect body odours, to preserve the ecoflora of the armpits, of the auditory canals, of the mammary areolae and of the perianal regions where most of the apocrine sweat glands occur. There also exists a need to have available novel compositions which are effective in preventing and/or treating body odours and which are pleasant and comfortable to employ.

An object of the present invention is to satisfy these needs.

GB 2 262 887 discloses a deodorant composition in the form of a roll-on or stick comprising an antimicrobial mixture composed of a coriander essential oil and of a lichen extract. Coriander essential oils generally do not comprise monounsaturated fatty acid.

The inventors have discovered, surprisingly, an activity with regard to body odours with a monounsaturated fatty acid or one of its salts and/or of its esters and/or of its amides or an oil comprising at least 40% by weight of the said fatty acid, so that it may be of use in a deodorant cosmetic composition. This is all the more surprising as monounsaturated fatty acids or oils enriched with these monounsaturated fatty acids do not have a detectable antibacterial activity with regard to the microorganisms responsible for unpleasant body odours, such as *Corynebacterium striatum, Corynebacterium mucifaciens* or *Corynebacterium xerosis.*

Consequently, a subject-matter of the present invention is the cosmetic use, as deodorant active agent, of a monounsaturated fatty acid or one of its salts and/or one of its esters and/or one of its amides, in particular in a composition comprising a physiologically acceptable medium.

It also relates to a cosmetic method by the oral route for treating and/or preventing unpleasant human body odours, consisting in ingesting a composition comprising, in a physiologically acceptable medium, at least one monounsaturated fatty acid or one of its salts and/or one of its esters and/or one of its amides.

It also relates to a cosmetic method by the topical route for treating and/or preventing unpleasant human body odours, consisting in applying, to the surface of a human keratinous substance, a composition comprising, in a physiologically acceptable medium, at least one monounsaturated fatty acid or one of its salts and/or one of its esters and/or one of its amides.

The term "deodorant active agent" is understood to mean, in the context of the present invention, any active agent which, by itself alone, has the effect of masking, absorbing, improving and/or reducing the unpleasant odour resulting from the decomposition of human sweat.

Within the meaning of the present invention, the term "physiologically acceptable medium" is understood to denote a cosmetically or dermatologically acceptable medium, that is to say a medium devoid of odour or unpleasant appearance and which is completely compatible with the oral or topical administration route.

In the case where the composition is intended for topical administration, that is to say for administration by application at the surface of the keratinous substance under consideration, such a medium is considered in particular to be physiologically acceptable when it does not cause stinging, tightness or redness unacceptable to the user.

Within the meaning of the present invention, the term "to prevent" is understood to mean the fact of reducing to a lesser degree the risk or the probability of the appearance of a given phenomenon, i.e., in the present invention, the release of unpleasant body odours.

### MONOUNSATURATED FATTY ACIDS

Within the meaning of the present invention, the term "monounsaturated fatty acid" is understood to mean a fatty acid, the hydrocarbon chain of which comprises a single double bond, and which is provided in a free form.

They are more particularly fatty acids having long hydrocarbon chains. The monounsaturated fatty acids which are suitable for the invention are in particular monounsaturated fatty acids comprising a hydrocarbon chain comprising from 12 to 22 carbon atoms.

The monounsaturated fatty acids which are suitable for the invention can be used in the acid form or in the salt form or also in the form of derivatives, in particular of fatty acid esters and amides.

When they are provided in the form of salts, the monounsaturated fatty acids of the invention are more particularly cosmetically acceptable salts, that is to say, inorganic salts, such as ammonium salts, salts of alkali metals, such as lithium, potassium or sodium, salts of alkaline earth metals, such as magnesium or calcium, or aluminium salts.

In particular, the monounsaturated fatty acids which are suitable for the invention can be provided in the form of calcium salts.

When they are provided in the form of esters, the monounsaturated fatty acids of the invention can be esterified with glycerol in the mono-, di- or triacyl form, with an alcohol, such as methyl alcohol and ethyl alcohol, with a sugar, in particular a monosaccharide or a disaccharide, a tocopherol, a tocotrienol, a sterol, such as cholesterol or a phytosterol, such as β-sitosterol, or with a fatty alcohol, in particular a C₈ to C₁₈ fatty alcohol.

It is understood that the choice of the monounsaturated fatty acids of the invention is made taking into account the end use of the composition comprising them.

The monounsaturated fatty acid of the invention, its salt and/or its ester can be employed in a composition by the oral route in which the content of the said monounsaturated fatty acid, its salt and/or its ester is such that the daily dose varies from 0.5 to 2500 mg/d, in particular from 1 to 2000 mg/d, more particularly from 2 to 1500 mg/d, indeed even from 3 to 1000 mg/d and in particular from 5 to 600 mg/d.

Among the monounsaturated fatty acids which are suitable for the invention, use may more particularly be made of oleic acid, petroselinic acid, sapienic acid, *cis*-8-octadecenoic acid or *cis*-vaccenic acid, or their mixtures. Petroselinic acid is very particularly suitable for the invention.

According to one alternative form of the invention, the monounsaturated fatty acid or acids are used in an isolated form, that is to say, after extraction from their source of origin.

According to another alternative form of the invention, the monounsaturated fatty acid or acids are used in a plant extract, such as an oil.

Thus, the invention relates in particular to the cosmetic use of an oil rich in monounsaturated fatty acid of the invention and especially of an oil rich in petroselinic acid.

The term "oil rich in monounsaturated fatty acid of the invention" is understood to mean an oil comprising at least 40% by weight of monounsaturated fatty acid, and preferably from 40 to 95% by weight, and more preferably from 65 to 95% by weight of monounsaturated fatty acid of its salt and/or of its ester and/or its amide.

The oils rich in petroselinic acid are more particularly chosen from umbellifer oils.

The term "oil rich in petroselinic acid" is understood to mean an oil comprising at least 40% by weight of petroselinic acid, and preferably from 40 to 95% by weight, and more preferably from 65 to 95% by weight of petroselinic acid.

Umbellifers are plants having flowers arranged in umbels; the species which are particularly rich in petroselinic acid are the *Umbelliferae, Apiaceae* and *Araliaceae.*

The plants of the genus *Thapsia* are also sources of petroselinic acid (Avato et al., Lipids, 2001, 36, 845).

The species preferably used in the invention are coriander, chervil, carrot, celery, cumin, caraway, parsley and dill, or their mixtures.

The umbellifer oil used according to the invention can be extracted from the seed of an umbellifer, for example by grinding or pressing, followed by refining.

The umbellifer oil has a petroselinic acid content which varies according to the umbellifer seed from which it is extracted. For one and the same umbellifer, the petroselinic acid content also varies according to the country of origin of the umbellifer and according to the extraction, which may be more or less complete.

Petroselinic acid is also an abundant compound (approximately 48% by weight) of the seed oil of *Geranium sanguineum* (Tsevegsuren et al., Lipids, 2004, 39, 571).

According to one embodiment, the monounsaturated fatty acid more particularly under consideration in the invention is petroselinic acid.

In particular, the petroselinic acid can be used in the form of an umbellifer oil or a *Geranium sanguineum* oil comprising at least 40% by weight of petroselinic acid, and preferably from 40 to 95% by weight, and more preferably from 65 to 95% by weight of petroselinic acid.

According to another embodiment, the umbellifer oil more particularly under consideration in the invention can be chosen from the seed oils of coriander, chervil, carrot, celery, cumin, caraway, parsley and dill, and their mixtures.

### FORMULATION FORMS

### I/ ORAL ROUTE

The compositions according to the invention can be administered orally. The compositions according to the invention can be provided in any formulation form normally used according to the oral route.

A composition according to the invention comprises a physiologically acceptable medium.

The term "composition by the oral route" is understood to mean, for example, nutritional, nutraceutical or cosmeceutical compositions comprising at least one monounsaturated fatty acid.

In the case of a composition suitable for oral administration, the use of an ingestible support is favoured. The ingestible support can be of diverse nature according to the type of composition under consideration.

For ingestion, numerous embodiments of oral compositions and in particular of food supplements are possible.

The formulation of such compositions can be carried out by any usual process known to a person skilled in the art for producing, for example, solutions to be taken orally, sugar-coated tablets, hard gelatin capsules, gels, emulsions, tablets to be swallowed or chewed, capsules, in particular soft or hard capsules, granules to be dissolved, syrups, solid or liquid foods and hydrogels which make possible controlled release.

Tablets, gels or lozenges, suspensions, oral supplements in the dry form and oral supplements in the liquid form are suitable, for example, as food support.

In particular, an active agent according to the invention can be incorporated into any form of food supplement or enriched food, for example food bars or compact or loose powders. The powders can be diluted with water, in soda, dairy products or soybean derivatives, or can be incorporated into food bars.

According to a preferred embodiment, a composition according to the invention administered orally can be formulated in the form of a sugar-coated tablet, hard gelatin capsule, gel, emulsion, tablet, capsule, hydrogel, food bar, compact or loose powder, liquid suspension or solution, confectionery product, fermented milk, fermented cheese, chewing gum, toothpaste or spray solution.

Milk, yoghurt, cheese, fermented milks, milk-based fermented products, ice creams, products based on fermented or non-fermented cereals, milk-based powders, infant and baby formulae, animal feed, in particular for pets, tablets or lozenges, liquid bacterial suspensions, oral supplements in the dry form and oral supplements in the liquid form are suitable, for example, as food supports.

The compositions by the oral route can be provided either in the anhydrous form or in the aqueous form according to the dermocosmetic indication.

An active agent according to the invention can be formulated with the usual excipients and components for such oral compositions or food supplements, namely in particular fatty and/or aqueous components, humectants, thickeners, preservatives, texturing, flavouring and/or coating agents, antioxidants and dyes which are normal in the food sector.

The formulating agents and excipients for oral compositions, in particular for food supplements, are known in this field and are not the subject of a detailed description here.

In particular, the composition according to the invention can be a food composition for human consumption. They can in particular be nutritional complete foods, drinks, mineral waters, soups, dietary supplements and replacement or substitute foods, nutritional bars, confectionery products, milk-based products or fermented-milk-based products, yoghurts, milk-based powders, enteral nutritional products, infant and/or baby compositions, products based on fermented or non-fermented cereals, ice creams, chocolate, coffee or "culinary" products, such as mayonnaise, tomato puree or salad dressings.

### Process

According to another of its aspects, the present invention relates to an oral cosmetic treatment method for the treatment of body odours which can in particular be carried out by administering the cosmetic compositions as defined above, according to the usual technique for the use of these compositions.

According to one embodiment, the invention relates to an oral cosmetic method for preventing and/or treating body odours in an individual having need thereof, comprising at least one stage of administering to the said individual, as active agent, at least one oil comprising a monounsaturated fatty acid, or one monounsaturated fatty acid or one of its salts and/or of its esters.

A cosmetic method according to the invention can be implemented in particular by administering a food composition as defined above.

A method of the invention can be implemented on a daily basis, for example, at a rate, for example, of a single administration per day or of an administration twice a day, for example once in the morning and once in the evening, or three times a day, in particular at each meal.

A cosmetic method according to the invention can be implemented, for example, by daily administration of a composition formulated, for example, in the form of hard gelatin capsules, sugar-coated tablets, emulsions, tablets, capsules or phials to be taken orally, in appropriate amount and appropriate number, depending on their form.

An effective amount of monounsaturated fatty acid can be administered in a single dose per day or in doses split up over the day, for example two to three times a day.

A method according to the invention can advantageously comprise a single administration.

### II/ TOPICAL ROUTE

The compositions according to the invention can be administered topically to the surface of human keratinous substances comprising the skin, hair, scalp and mucous membranes. The compositions according to the invention can be provided in any formulation form normally used.

The composition can also comprise, in addition to the monounsaturated fatty acid, at least one additional deodorant active agent as defined below and/or one antiperspirant active agent as defined below.

### Deodorant active agents

The composition according to the invention can comprise one or more deodorant active agents, such as, for example:
- bacteriostatic agents or other bactericidal agents, such as 2,4,4'-trichloro-2'-hydroxydiphenyl ether (triclosan), 2,4-dichloro-2'-hydroxydiphenyl ether, 3',4',5'-trichlorosalicylanilide, 1-(3',4'-dichlorophenyl)-3-(4'-chlorophenyl)urea (triclocarban) or 3,7,11-trimethyldodeca-2,5,10-trienol (farnesol); quaternary ammonium salts, such as cetyltrimethylammonium salts or cetylpyridinium salts; chlorhexidine and its salts; diglyceryl monocaprate, diglyceryl monolaurate, glyceryl monolaurate; polyhexamethylene biguanide salts;
- zinc salts, such as zinc salicylate, zinc phenolsulfonate, zinc pyrrolidonecarboxylate (more commonly known as zinc pidolate), zinc sulfate, zinc chloride, zinc lactate, zinc gluconate, zinc ricinoleate, zinc glycinate, zinc carbonate, zinc citrate, zinc chloride, zinc laurate, zinc oleate, zinc orthophosphate, zinc stearate, zinc tartrate, zinc lactate, zinc acetate or their mixtures;
- odour absorbers, such as zeolites, cyclodextrins, metal oxide silicates, such as those described in Application US 2005/063928, metal oxide particles modified by a transition metal, such as described in Applications US 2005/084464 and US 2005/084474, aluminosilicates, such as those described in Application EP 1 658 863, or particles of chitosan derivatives, such as those described in Patent US 6 916 465;
- substances which block the enzymatic reactions responsible for the formation of odorous compounds, such as arylsulfatase, 5-lipoxygenase, aminocylase or β-glucuronidase inhibitors and their mixtures.

The deodorant active agents can be present in the composition according to the invention in a proportion of from 0.01% to 10% by weight and preferably in a proportion of from 0.1% to 5% by weight, with respect to the total weight of the composition.

### Antiperspirant active agents

The term "antiperspirant active agent" is understood to mean any substance which, by itself alone, has the effect of reducing the flow of sweat, of reducing the sensation on the skin of moisture associated with human sweat and of masking human sweat.

The antiperspirant active agents are preferably chosen from aluminium and/or zirconium salts; complexes of zirconium hydroxychloride and of aluminium hydroxychloride with an amino acid, such as those described in Patent US-3 792 068, commonly known as "ZAG" complexes. Such complexes are generally known under the name ZAG (when the amino acid is glycine). The ZAG complexes ordinarily exhibit an Al/Zr quotient ranging from approximately 1.67 to 12.5 and a metal/CI quotient ranging from approximately 0.73 to 1.93. Mention may be made, among these products, of aluminium zirconium octachlorohydrex GLY, aluminium zirconium pentachlorohydrex GLY, aluminium zirconium tetrachlorohydrate GLY and aluminium zirconium trichlorohydrate GLY.

Mention may be made, among the aluminium salts, of aluminium chlorohydrate, aluminium chlorohydrex, aluminium chlorohydrex PEG, aluminium chlorohydrex PG, aluminium dichlorohydrate, aluminium dichlorohydrex PEG, aluminium dichlorohydrex PG, aluminium sesquichlorohydrate, aluminium sesquichlorohydrex PEG, aluminium sesquichlorohydrex PG, alum salts, aluminium sulfate, aluminium zirconium octachlorohydrate, aluminium zirconium pentachlorohydrate, aluminium zirconium tetrachlorohydrate, aluminium zirconium trichlorohydrate and more particularly the aluminium hydroxychloride sold by Reheis under the name Reach 301 or by Guilini Chemie under the name Aloxicoll PF 40. Aluminium zirconium salts are, for example, the salt sold by Reheis under the name Reach AZP-908-SUF.
Use will more particularly be made of aluminium chlorohydrate in the activated or non-activated form.

The antiperspirant active agents can be present in the composition according to the invention in a proportion of from 0.001% to 30% by weight and preferably in a proportion of from 0.5% to 25% by weight, with respect to the total weight of the composition.

The composition according to the invention can be provided in any formulation form conventionally used for a topical application and in particular in the form of aqueous gels or of aqueous or aqueous/alcoholic solutions. They can also, by addition of a fatty or oily phase, be provided in the form of dispersions of the lotion type, of emulsions with a liquid or semi-liquid consistency of the milk type, obtained by dispersion of a fatty phase in an aqueous phase (O/W) or vice versa (W/O), or of suspensions or emulsions with a soft, semi-solid or solid consistency of the cream or gel type, or alternatively of multiple emulsions (W/O/W or O/W/O), of microemulsions, of vesicular dispersions of ionic and/or non-ionic type, or of wax/aqueous phase dispersions. These compositions are prepared according to the usual methods.

The compositions can in particular be packaged in the pressurized form in an aerosol device or in a pump-action spray; packaged in a device equipped with an openwork wall, in particular a grating; packaged in a device equipped with a ball applicator ("roll-on"); packaged in the form of sticks or packaged in the form of a loose or compacted powder. In this regard, they comprise the ingredients generally used in products of this type, which are well known to a person skilled in the art.

According to another specific form of the invention, the compositions according to the invention can be anhydrous.

The term "anhydrous composition" is understood to mean a composition comprising less than 2% by weight of water, indeed even less than 0.5% of water, and in particular devoid of water, the water not being added during the preparation of the composition but corresponding to the residual water contributed by the mixed ingredients.

According to another specific form of the invention, the compositions according to the invention can be solid, in particular in the stick form.

The term "solid composition" is understood to mean that the measurement of the maximum force measured by texturometry while driving a probe into the formula sample must be at least equal to 0.25 newton, in particular at least equal to 0.30 newton and especially at least equal to 0.35 newton, assessed under precise measurement conditions as follows.

The formulae are poured hot into jars with a diameter of 4 cm and a depth of 3 cm. Cooling is carried out at ambient temperature. The hardness of the formulae produced is measured after an interval of 24 hours. The jars containing the samples are characterized in texturometry using a texture analyzer, such as that sold by Rheo, TA-XT2, according to the following protocol: a probe of stainless-steel ball type with a diameter of 5 mm is brought into contact with the sample at a rate of 1 mm/s. The measurement system detects the interface with the sample, with a detection threshold equal to 0.005 newton. The probe sinks 0.3 mm into the sample, at a rate of 0.1 mm/s. The measuring device records the change in the force measured in compression over time, during the penetration phase. The hardness of the sample corresponds to the mean of the maximum values of the force detected during the penetration, over at least three measurements.

### AQUEOUS PHASE

The compositions according to the invention intended for the cosmetic use can comprise at least one aqueous phase. They are formulated in particular as aqueous lotions or as water-in-oil or oil-in-water emulsions or as multiple emulsions (oil-in-water-in-oil or water-in-oil-in-water triple emulsions) (such emulsions are known and are described, for example, by C. Fox in "Cosmetics and Toiletries", November 1986, Vol. 101, pages 101-112).

The aqueous phase of the said compositions comprises water and generally other water-soluble or water-miscible solvents. The water-soluble or water-miscible solvents comprise short-chain, for example C₁-C₄, monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol. Use will more particularly be made of propylene glycol and glycerol, propane-1,3-diol.

The composition according to the invention preferably has a pH ranging from 3 to 9, according to the support chosen.

### EMULSIFIERS

### Oil-in-water emulsifiers

Mention may be made, as emulsifiers which can be used in the oil-in-water emulsions or oil-in-water-in-oil triple emulsions, for example, of non-ionic emulsifiers, such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids; oxyalkylenated (oxyethylenated and/or oxypropylenated) ethers of fatty alcohols; sugar esters, such as sucrose stearate; and their mixtures, such as the mixture of glyceryl stearate and PEG-40 stearate.

Mention may also be made of fatty alcohol/alkylpolyglycoside emulsifying mixtures, such as are described in Applications WO 92/06778, WO 95/13863 and WO 98/47610, such as the commercial products sold by Seppic under the name Montanov®.

### Water-in-oil emulsifiers

Mention may be made, among the emulsifiers which can be used in the water-in-oil emulsions or water-in-oil-in-water triple emulsions, by way of example, of alkyl dimethicone copolyols corresponding to the following formula (I): 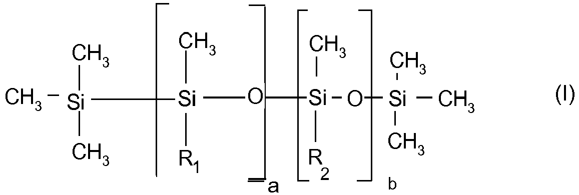 in which:
R₁ denotes a linear or branched C₁₂-C₂₀ and preferably C₁₂-C₁₈ alkyl group;
R₂ denotes the group: --CₙH₂ₙ--(-OC₂H₄-)ₓ--(-OC₃H₆-)_{y}--O-R₃;
R₃ denotes a hydrogen atom or a linear or branched alkyl radical comprising from 1 to 12 carbon atoms;
a is an integer ranging from 1 to approximately 500;
b denotes an integer ranging from 1 to approximately 500;
n is an integer ranging from 2 to 12 and preferably from 2 to 5;
x denotes an integer ranging from 1 to approximately 50 and preferably from 1 to 30;
y denotes an integer ranging from 0 to approximately 49 and preferably from 0 to 29, with the proviso that, when y is other than zero, the ratio x/y is greater than 1 and preferably varies from 2 to 11.

Mention will more particularly be made, among the preferred alkyl dimethicone copolyol emulsifiers of formula (I), of Cetyl PEG/PPG-10/1 Dimethicone and more particularly the mixture Cetyl PEG/PPG-10/1 Dimethicone and Dimethicone (INCI name), such as the product sold under the trade name Abil EM90 by Goldschmidt, or alternatively the mixture (Polyglyceryl-4 Stearate and Cetyl PEG/PPG-10 (and) Dimethicone (and) Hexyl Laurate), such as the product sold under the trade name Abil WE09 by the same company.

Mention may also be made, among the water-in-oil emulsifiers, of the dimethicone copolyols corresponding to the following formula (II): 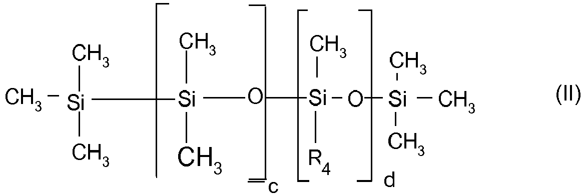 in which:
R₄ denotes the group: --CₘH₂ₘ--(-OC₂H₄-)ₛ--(-OC₃H₆-)ₜ--O-R₅,
R₅ denotes a hydrogen atom or a linear or branched alkyl radical comprising from 1 to 12 carbon atoms;
c is an integer ranging from 1 to approximately 500;
d denotes an integer ranging from 1 to approximately 500;
m is an integer ranging from 2 to 12 and preferably from 2 to 5;
s denotes an integer ranging from 1 to approximately 50 and preferably from 1 to 30;
t denotes an integer ranging from 0 to approximately 50 and preferably from 0 to 30; with the proviso that the sum s+t is greater than or equal to 1.

Use will particularly be made, among these preferential dimethicone copolyol emulsifiers of formula (II), of PEG-18/PPG-18 Dimethicone and more particularly the mixture Cyclopentasiloxane (and) PEG-18/PPG-18 Dimethicone (INCI name), such as the product sold by Dow Corning under the trade name Silicone DC 5225 C or KF-6040 from Shin-Etsu.

According to a particularly preferred form, use will be made of a mixture of at least one emulsifier of formula (I) and of at least one emulsifier of formula (II).

Use will be made more particularly of a mixture of PEG-18/PPG-18 Dimethicone and Cetyl PEG/PPG-10/1 Dimethicone and even more particularly of a mixture of (Cyclopentasiloxane (and) PEG-18/PPG-18 Dimethicone) and of Cetyl PEG/PPG-10/1 Dimethicone and Dimethicone or of (Polyglyceryl-4 Stearate and Cetyl PEG/PPG-10 (and) Dimethicone (and) Hexyl Laurate).

Mention will also be made, among the water-in-oil emulsifiers, of non-ionic emulsifiers derived from fatty acids and polyols, alkyl polyglycosides (APGs), sugar esters and their mixtures.

Use may in particular be made, as non-ionic emulsifiers derived from fatty acids and polyols, of fatty acid esters of polyols, the fatty acid having in particular a C₈-C₂₄ alkyl chain and the polyols being, for example, glycerol and sorbitan.

Mention may in particular be made, as fatty acid esters of polyols, of isostearic acid esters of polyols, stearic acid esters of polyols, and their mixtures, in particular isostearic acid esters of glycerol and/or sorbitan.

Mention may in particular be made, as stearic acid esters of polyols, of the polyethylene glycol esters, such as PEG-30 Dipolyhydroxystearate, for example the product sold under the name Arlacel P135 by ICI.

Mention may be made, as glycerol and/or sorbitan esters, for example, of polyglyceryl isostearate, such as the product sold under the name Isolan Gl 34 by Goldschmidt; sorbitan isostearate, such as the product sold under the name Arlacel 987 by ICI; sorbitan glyceryl isostearate, such as the product sold under the name Arlacel 986 by ICI, the mixture of sorbitan isostearate and polyglyceryl isostearate (3 mol) sold under the name Arlacel 1690 by Uniqema, and their mixtures.

The emulsifier can also be chosen from alkylpolyglycosides having an HLB of less than 7, for example those represented by the following general formula (1):

R-O-(G)ₓ (1)

in which R represents a branched and/or unsaturated alkyl radical comprising from 14 to 24 carbon atoms, G represents a reduced sugar comprising 5 or 6 carbon atoms and x denotes a value ranging from 1 to 10 and preferably from 1 to 4, and G in particular denotes glucose, fructose or galactose.

The unsaturated alkyl radical can comprise one or more ethylenic unsaturations and in particular one or two ethylenic unsaturations.

Mention may be made, as alkyl polyglycosides of this type, of alkyl polyglucosides (G = glucose in the formula (I)) and in particular the compounds of formula (I) in which R more particularly represents an oleyl radical (unsaturated C₁₈ radical) or isostearyl (saturated C₁₈ radical), G denotes glucose and x is a value ranging from 1 to 2, in particular isostearyl glucoside, oleyl glucoside and their mixtures. This alkyl polyglucoside can be used as a mixture with a coemulsifier, more especially with a fatty alcohol and in particular a fatty alcohol having the same fatty chain as that of the alkyl polyglucoside, that is to say comprising from 14 to 24 carbon atoms and having a branched and/or unsaturated chain, for example isostearyl alcohol when the alkyl polyglucoside is isostearyl glucoside and oleyl alcohol when the alkyl polyglucoside is oleyl glucoside, optionally in the form of a self-emulsifying composition, as described, for example, in the document WO-A-92/06778. Use may be made, for example, of the mixture of isostearyl glucoside and isostearyl alcohol, sold under the name Montanov WO 18 by Seppic, and also the mixture of octyldodecanol and octyldodecyl xyloside sold under the name Fludanov 20X by Seppic.

Mention may also be made of succinic-terminated polyolefins, such as esterified succinic-terminated polyisobutylenes and their salts, in particular the diethanolamine salts, such as the products sold under the names Lubrizol 2724, Lubrizol 2722 and Lubrizol 5603 by Lubrizol or the commercial product Chemcinnate 2000.

The total amount of emulsifiers in the composition will preferably be, in the composition according to the invention, at active material contents ranging from 1% to 8% by weight and more particularly from 2% to 6% by weight, with respect to the total weight of the composition.

### FATTY PHASE

The compositions according to the invention can comprise at least one water-immiscible organic liquid phase, known as fatty phase. This phase generally comprises one or more hydrophobic compounds which render the said phase water-immiscible. The said phase is liquid (in the absence of structuring agent) at ambient temperature (20-25°C). Preferentially, the water-immiscible organic liquid phase in accordance with the invention generally comprises at least one volatile oil and/or at least one non-volatile oil and optionally at least one structuring agent.

The term "oil" is understood to mean a fatty substance which is liquid at ambient temperature (25°C) and atmospheric pressure (760 mmHg, i.e. 10⁵ Pa). The oil can be volatile or non-volatile.

Within the meaning of the invention, the term "volatile oil" is understood to mean an oil which is capable of evaporating on contact with the skin or with the keratinous fibre in less than one hour, at ambient temperature and atmospheric pressure. The volatile oils of the invention are volatile cosmetic oils which are liquid at ambient temperature and which have a non-zero vapour pressure, at ambient temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is understood to mean an oil which remains on the skin or the keratinous fibre at ambient temperature and atmospheric pressure for at least several hours and which has in particular a vapour pressure of less than 10⁻³ mmHg (0.13 Pa).

The oil can be chosen from any physiologically acceptable oil and in particular cosmetically acceptable oil, especially mineral, animal, vegetable or synthetic oils; in particular volatile or non-volatile hydrocarbon oils and/or silicone oils and/or fluorinated oils, and their mixtures.

More specifically, the term "hydrocarbon oil" is understood to mean an oil mainly comprising carbon and hydrogen atoms and optionally one or more functional groups chosen from hydroxyl, ester, ether and carboxylic acid functional groups. Generally, the oil exhibits a viscosity of 0.5 to 100 000 mPa.s, preferably of 50 to 50 000 mPa.s and more preferably of 100 to 30 000 mPa.s.

Mention may be made, as examples of volatile oil which can be used in the invention, of:
- volatile hydrocarbon oils chosen from hydrocarbon oils having from 8 to 16 carbon atoms, in particular C₈-C₁₆ isoalkanes of petroleum origin (also known as isoparaffins), such as isododecane (also known as 2,2,4,4,6-pentamethylheptane), isodecane or isohexadecane, for example the oils sold under the Isopar or Permetyl trade names, branched C₈-C₁₆ esters, isohexyl neopentanoate, and their mixtures. Use may also be made of other volatile hydrocarbon oils, such as petroleum distillates, in particular those sold under the name Shell Solt by Shell; and volatile linear alkanes, such as those described in Patent Application DE10 2008 012 457 from Cognis;
- volatile silicones, such as, for example, volatile linear or cyclic silicone oils, in particular those having a viscosity ≤ 8 centistokes (8×10⁻⁶ m²/s) and having in particular from 2 to 7 silicon atoms, these silicones optionally comprising alkyl or alkoxy groups having from 1 to 10 carbon atoms. Mention may in particular be made, as volatile silicone oils which can be used in the invention, of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane or dodecamethylpentasiloxane;
- and their mixtures.

Mention may also be made of the volatile linear alkyltrisiloxane oils of general formula (I): 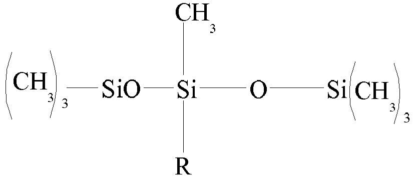 where R represents an alkyl group comprising from 2 to 4 carbon atoms, one or more hydrogen atoms of which can be replaced by a fluorine or chlorine atom.

Mention may be made, among the oils of general formula (I), of:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
   corresponding to the oils of formula (I) for which R is, respectively, a butyl group, a propyl group or an ethyl group.

Mention may be made, as examples of non-volatile oil which can be used in the invention, of:
- hydrocarbon oils of animal origin, such as perhydrosqualene;
- vegetable hydrocarbon oils, such as liquid triglycerides of fatty acids having 4 to 24 carbon atoms, such as heptanoic or octanoic acid triglycerides, or else wheat germ oil, olive oil, sweet almond oil, palm oil, rapeseed oil, cottonseed oil, alfalfa oil, poppy oil, pumpkinseed oil, cucumber oil, blackcurrant oil, evening primrose oil, millet oil, barley oil, quinoa oil, rye oil, safflower oil, candlenut oil, passionflower oil, musk rose oil, sunflower oil, maize oil, soybean oil, grape seed oil, sesame oil, hazelnut oil, apricot oil, macadamia oil, castor oil, avocado oil, caprylic/capric acid triglycerides, such as those sold by Stéarineries Dubois or those sold under the names Miglyol 810, 812 and 818 by Dynamit Nobel, jojoba oil or shea butter oil;
- linear or branched hydrocarbons of mineral or synthetic origin, such as liquid paraffins and their derivatives, liquid petrolatum, polydecenes, polybutenes, hydrogenated polyisobutene, such as Parleam, or squalane;
- synthetic ethers having from 10 to 40 carbon atoms;
- synthetic esters, in particular of fatty acids, such as the oils of formula R₁COOR₂ in which R₁ represents the residue of a linear or branched higher fatty acid comprising from 1 to 40 carbon atoms and R₂ represents a hydrocarbon chain, in particular a branched hydrocarbon chain, comprising from 1 to 40 carbon atoms, with R₁ + R₂ ≥ 10, such as, for example, purcellin oil (cetearyl octanoate), isononyl isononanoate, isopropyl myristate, isopropyl palmitate, C₁₂-C₁₅ alkyl benzoate, hexyl laurate, diisopropyl adipate, isononyl isononanoate, 2-ethylhexyl palmitate, 2-octyldodecyl stearate, 2-octyldodecyl erucate, isostearyl isostearate or tridecyl trimellitate; octanoates, decanoates or ricinoleates of alcohols or polyalcohols, such as propylene glycol dioctanoate; hydroxylated esters, such as isostearyl lactate, octyl hydroxystearate, octyldodecyl hydroxystearate, diisostearyl malate, triisocetyl citrate or heptanoates, octanoates or decanoates of fatty alcohols; polyol esters, such as propylene glycol dioctanoate, neopentyl glycol diheptanoate or diethylene glycol diisononanoate; and pentaerythritol esters, such as pentaerythrityl tetraisostearate;
- fatty alcohols which are liquid at ambient temperature and which comprise a branched and/or unsaturated carbon-based chain having from 12 to 26 carbon atoms, such as octyldodecanol, isostearyl alcohol, 2-butyloctanol, 2-hexyldecanol, 2-undecylpentadecanol or oleyl alcohol;
- higher fatty acids, such as oleic acid, linoleic acid or linolenic acid;
- carbonates;
- acetates;
- citrates;
- fluorinated oils which are optionally partially hydrocarbon-based and/or silicone-based, for example fluorosilicone oils, fluoropolyethers or fluorinated silicones, such as described in the document EP-A-847 752;
- silicone oils, such as non-volatile linear or cyclic polydimethylsiloxanes (PDMSs); polydimethylsiloxanes comprising alkyl, alkoxy or phenyl groups which are pendent or at the end of the silicone chain, which groups have from 2 to 24 carbon atoms; or phenylated silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates, and
- their mixtures.

### Structuring agent

The compositions according to the invention comprising a fatty phase can additionally comprise at least one structuring agent for the said fatty phase, which can preferably be chosen from waxes, pasty compounds, inorganic or organic lipophilic gelling agents, and their mixtures.

It is understood that the amounts of these compounds can be adjusted by a person skilled in the art so as not to harm the effect desired in the context of the present invention.

### Wax(es)

The wax is generally a lipophilic compound which is solid at ambient temperature (25°C), which exhibits a reversible solid/liquid change in state and which has a melting point of greater than or equal to 30°C which can range up to 200°C and in particular up to 120°C.

In particular, the waxes suitable for the invention can exhibit a melting point of greater than or equal to 45°C and in particular of greater than or equal to 55°C.

Within the meaning of the invention, the melting point corresponds to the temperature of the most endothermic peak observed in thermal analysis (DSC) as described in Standard ISO 11357-3; 1999. The melting point of the wax can be measured using a differential scanning calorimeter (DSC), for example the calorimeter sold under the name MDSC 2920 by TA Instruments.

The measurement protocol is as follows:
A sample of 5 mg of wax placed in a crucible is subjected to a first temperature rise ranging from -20°C to 100°C, at a heating rate of 10°C/minute, is then cooled from 100°C to -20°C at a cooling rate of 10°C/minute and is finally subjected to a second temperature rise ranging from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the value of the temperature corresponding to the tip of the peak of the curve representing the variation in the difference in power absorbed as a function of the temperature.

The waxes capable of being used in the compositions according to the invention are chosen from waxes which are solid at ambient temperature and which are of animal, vegetable, mineral or synthetic origin, and their mixtures.

Mention may in particular be made, by way of illustration of the waxes which are suitable for the invention, of hydrocarbon waxes, such as beeswax, lanolin wax, Chinese insect waxes, rice bran wax, carnauba wax, candelilla wax, ouricury wax, esparto wax, berry wax, shellac wax, Japan wax and sumac wax; montan wax, orange wax and lemon wax, refined sunflower wax, sold under the name Sunflower Wax by Koster Keunen, microcrystalline waxes, paraffin waxes and ozokerite; polyethylene waxes, the waxes obtained by the Fischer-Tropsch synthesis and waxy copolymers, and also their esters.

Mention may also be made of waxes obtained by catalytic hydrogenation of animal or vegetable oils having linear or branched C₈-C₃₂ fatty chains. Mention may in particular be made, among these waxes, of isomerized jojoba oil, such as the trans-isomerized partially hydrogenated jojoba oil manufactured or sold by Desert Whale under the commercial reference Iso-Jojoba-50®, hydrogenated sunflower oil, hydrogenated castor oil, hydrogenated coconut oil, hydrogenated lanolin oil and bis(1,1,1-trimethylolpropane) tetrastearate, sold under the name Hest 2T-4S® by Heterene.

Mention may also be made of silicone waxes (C₃₀₋₄₅ alkyl dimethicone) or fluorinated waxes.

Use may also be made of the waxes obtained by hydrogenation of castor oil esterified with cetyl alcohol, sold under the names Phytowax Castor 16L64® and 22L73® by Sophim. Such waxes are described in Application FR-A-2 792 190.

Use may be made, as wax, of a C₂₀-C₄₀ alkyl (hydroxystearyloxy)stearate (the alkyl group comprising from 20 to 40 carbon atoms), alone or as a mixture.

Such a wax is sold in particular under the names Kester Wax K 82 P®, Hydroxypolyester K 82 P® and Kester Wax K 80 P® by Koster Keunen.

Mention may in particular be made, as microwaxes which can be used in the compositions according to the invention, of carnauba microwaxes, such as that sold under the name MicroCare 350® by Micro Powders, synthetic wax microwaxes, such as that sold under the name MicroEase 114S® by Micro Powders, microwaxes composed of a mixture of carnauba wax and polyethylene wax, such as those sold under the names Micro Care 300® and 310® by Micro Powders, microwaxes composed of a mixture of carnauba wax and synthetic wax, such as that sold under the name Micro Care 325® by Micro Powders, polyethylene microwaxes, such as those sold under the names Micropoly 200®, 220®, 220L® and 250S® by Micro Powders, the commercial products Performalene 400 Polyethylene and Performalene 500-L Polyethylene from New Phase Technologies, Performalene 655 Polyethylene or paraffin waxes, such as the wax having the INCI name Microcrystalline Wax and Synthetic Wax and sold under the trade name Microlease by Sochibo; polytetrafluoroethylene microwaxes, such as those sold under the names Microslip 519® and 519 L® by Micro Powders.

The composition according to the invention will preferably comprise a content of wax(es) ranging from 3% to 20% by weight, in particular from 5% to 15% by weight and more particularly from 6% to 15% by weight, with respect to the total weight of the composition.

According to a specific form of the invention, in the context of anhydrous solid compositions in the stick form, use will be made of polyethylene microwaxes in the form of crystallites with an aspect ratio at least equal to 2 and with a melting point ranging from 70°C to 110°C and preferably from 70°C to 100°C, in order to reduce or indeed even eliminate the presence of strata in the solid composition.

These crystallites in needle form and in particular their dimensions can be characterized visually according to the following method.

The wax is deposited on a microscope slide, which is placed on a hotplate. The slide and the wax are heated to a temperature generally at least 5°C higher than the melting point of the wax or of the mixture of waxes under consideration. At the end of melting, the liquid thus obtained and the microscope slide are allowed to cool in order to solidify. Observation of the crystallites is performed using a Leica DMLB100 optical microscope, with an objective lens selected as a function of the size of the objects to be viewed, and under polarized light. The dimensions of the crystallites are measured using image analysis software, such as that sold by Microvision.

The crystallite polyethylene waxes in accordance with the invention preferably have an average length ranging from 5 to 10 µm. The term "average length" denotes the dimension given by the statistical particle size distribution at half the population, referred to as D50.

Use will be made more particularly of a mixture of Performalene 400 Polyethylene and Performalene 500-L Polyethylene waxes from New Phase Technologies.

### Pasty compounds

Within the meaning of the present invention, the term "pasty compound" is understood to mean a lipophilic fatty compound which exhibits a reversible solid/liquid change in state, which exhibits, in the solid state, an anisotropic crystalline arrangement and which comprises, at a temperature of 23°C, a liquid fraction and a solid fraction.

The pasty compound is preferably chosen from synthetic compounds and compounds of vegetable origin. A pasty compound can be obtained by synthesis from starting materials of vegetable origin.

The pasty compound can advantageously be chosen from:
- lanolin and its derivatives,
- polymeric or non-polymeric silicone compounds,
- polymeric or non-polymeric fluorinated compounds,
- vinyl polymers, in particular:
- olefin homopolymers,
- olefin copolymers,
- hydrogenated diene homopolymers and copolymers,
- linear or branched and homo- or copolymeric oligomers of alkyl (meth)acrylates preferably having a C₈-C₃₀ alkyl group,
- homo- and copolymeric oligomers of vinyl esters having C₈-C₃₀ alkyl groups, and
- homo- and copolymeric oligomers of vinyl ethers having C₈-C₃₀ alkyl groups,
- liposoluble polyethers resulting from polyetherification between one or more C₂-C₁₀₀, preferably C₂-C₅₀, diols,
- esters,
- their mixtures.

Preference is given in particular, among the esters, to:
- esters of an oligomeric glycerol, in particular the esters of diglycerol, especially the condensates of adipic acid and of glycerol, for which a portion of the hydroxyl groups of the glycerols have reacted with a mixture of fatty acids, such as stearic acid, capric acid, stearic acid and isostearic acid and 12-hydroxystearic acid, such as in particular those sold under the Softisan 649 brand by Sasol,
- the arachidyl propionate sold under the Waxenol 801 brand by Alzo,
- phytosterol esters,
- fatty acid triglycerides and their derivatives,
- pentaerythritol esters,
- non-crosslinked polyesters resulting from the polycondensation between a linear or branched C₄-C₅₀ dicarboxylic acid or polycarboxylic acid and a C₂-C₅₀ diol or polyol,
- aliphatic esters of an ester resulting from the esterification of an aliphatic hydroxycarboxylic acid ester by an aliphatic carboxylic acid,
- polyesters resulting from the esterification, by a polycarboxylic acid, of an aliphatic hydroxycarboxylic acid ester, the said ester comprising at least two hydroxyl groups, such as the products Risocast DA-H® and Risocast DA-L®,
- esters of dimer diol and dimer diacid, if appropriate esterified on their free alcohol or acid functional group(s) by acid or alcohol radicals, such as Plandool-G,
- their mixtures.

The choice will preferably be made, among pasty compounds of vegetable origin, of a mixture of soya sterols and of oxyethylenated (5 EO)/oxypropylenated (5 PO) pentaerythritol sold under the reference Lanolide by Vevy.

### Lipophilic gelling agents

### Inorganic gelling agents

Mention may be made, as inorganic lipophilic gelling agent, of optionally modified clays, such as hectorites modified by a C₁₀ to C₂₂ ammonium chloride, such as hectorite modified by distearyldimethylammonium chloride, such as, for example, that sold under the name Bentone 38V® by Elementis.

Mention may also be made of fumed silica optionally hydrophobically treated at the surface, the size of the particles of which is less than 1 µm. This is because it is possible to chemically modify the surface of the silica by chemical reaction which results in a decrease in the number of silanol groups present at the surface of the silica. Silanol groups can in particular be replaced by hydrophobic groups: a hydrophobic silica is then obtained. The hydrophobic groups can be trimethylsiloxyl groups, which are obtained in particular by treatment of fumed silica in the presence of hexamethyldisilazane. Silicas thus treated are named "Silica silylate" according to the CTFA (8th edition, 2000). They are, for example, sold under the references Aerosil R812® by Degussa, Cab-O-Sil TS-530® by Cabot, dimethylsilyloxyl or polydimethylsiloxane groups, which are obtained in particular by treatment of fumed silica in the presence of polydimethylsiloxane or dimethyldichlorosilane. Silicas thus treated are named "Silica dimethyl silylate" according to the CTFA (8th edition, 2000). They are, for example, sold under the references Aerosil R972® and Aerosil R974® by Degussa and Cab-O-Sil TS-610® and Cab-O-Sil TS-720® by Cabot.

The hydrophobic fumed silica exhibits in particular a particle size which can be from nanometric to micrometric, for example ranging approximately from 5 to 200 nm.

### Organic gelling agents

The polymeric organic lipophilic gelling agents are, for example, partially or completely crosslinked organopolysiloxane elastomers with a three-dimensional structure, such as those sold under the names of KSG6®, KSG16® and KSG18® by Shin-Etsu, of Trefil E-505C® and Trefil E-506C® by Dow Corning, of Gransil SR-CYC®, SR DMF10®, SR-DC556®, SR 5CYC gel®, SR DMF 10 gel® and SR DC 556 gel® by Grant Industries and of SF 1204® and JK 113® by General Electric; ethylcellulose, such as that sold under the name Ethocel® by Dow Chemical; galactomannans comprising from one to six and in particular from two to four hydroxyl groups per monosaccharide and substituted by a saturated or unsaturated alkyl chain, such as guar gum alkylated by C₁ to C₆ and in particular C₁ to C₃ alkyl chains, and their mixtures; or block copolymers of "diblock", "triblock" or "radial" type of the polystyrene/polyisoprene or polystyrene/polybutadiene type, such as those sold under the name Luvitol HSB® by BASF, of the polystyrene/copoly(ethylene-propylene) type, such as those sold under the name Kraton® by Shell Chemical Co., or of the polystyrene/copoly(ethylene-butylene) type, or blends of triblock and radial (star) copolymers in isododecane, such as those sold by Penreco under the name Versagel®, such as, for example, the blend of butylene/ethylene/styrene triblock copolymer and of ethylene/propylene/styrene star copolymer in isododecane (Versagel M 5960).

Mention may also be made, as lipophilic gelling agent, of polymers with a weight-average molecular weight of less than 100 000 comprising a) a polymer backbone having hydrocarbon repeat units provided with at least one heteroatom and optionally b) at least one optionally functionalized pendent fatty chain and/or at least one optionally functionalized terminal fatty chain having from 6 to 120 carbon atoms and being bonded to these hydrocarbon units, such as described in Applications WO-A-02/056847 and WO-A-02/47619, in particular polyamide resins (especially comprising alkyl groups having from 12 to 22 carbon atoms), such as those described in US-A-5 783 657.

Mention may also be made, among the lipophilic gelling agents which can be used in the compositions according to the invention, of esters of dextrin and of fatty acid, such as dextrin palmitates, in particular such as those sold under the names Rheopearl TL® and Rheopearl KL® by Chiba Flour.

Use may also be made of silicone polyamides of the polyorganosiloxane type, such as those described in the documents US-A-5 874 069, US-A-5 919 441, US-A-6 051 216 and US-A-5 981 680.

These silicone polymers can belong to the following two families:
- polyorganosiloxanes comprising at least two groups capable of establishing hydrogen bond interactions, these two groups being situated in the chain of the polymer, and/or
- polyorganosiloxanes comprising at least two groups capable of establishing hydrogen bond interactions, these two groups being situated on grafts or branchings.

### Organic powder

According to a specific form of the invention, the compositions according to the invention will additionally comprise an organic powder.

In the present patent application, the term "organic powder" is understood to mean any solid which is insoluble in the medium at ambient temperature (25°C).

Mention may be made, as organic powders which can be used in the composition of the invention, for example, of polyamide particles and in particular those sold under the Orgasol names by Atochem; nylon 6,6 fibres, in particular the polyamide fibres sold by Etablissements P Bonte under the name Polyamide 0.9 Dtex 0.3 mm (INCI name: Nylon 6,6 or Polyamide 6,6) having a mean diameter of 6 µm, a weight of approximately 0.9 dtex and a length ranging from 0.3 mm to 1.5 mm; polyethylene powders; microspheres based on acrylic copolymers, such as those made of ethylene glycol dimethacrylate/lauryl methacrylate copolymer sold by Dow Corning under the name of Polytrap; polymethyl methacrylate microspheres, sold under the name Microsphere M-100 by Matsumoto or under the name Covabead LH85 by Wackherr; hollow polymethyl methacrylate microspheres (particle size: 6.5-10.5 µm) sold under the name Ganzpearl GMP 0800 by Ganz Chemical; methyl methacrylate/ethylene glycol dimethacrylate copolymer microbeads (size: 6.5-10.5 µm) sold under the name Ganzpearl GMP 0820 by Ganz Chemical or Microsponge 5640 by Amcol Health & Beauty Solutions; ethylene/acrylate copolymer powders, such as those sold under the name Flobeads by Sumitomo Seika Chemicals; expanded powders, such as hollow microspheres, in particular the microspheres formed of a terpolymer of vinylidene chloride, of acrylonitrile and of methacrylate sold under the name Expancel by Kemanord Plast under the references 551 DE 12 (particle size of approximately 12 µm and density of 40 kg/m³), 551 DE 20 (particle size of approximately 30 µm and density of 65 kg/m³) and 551 DE 50 (particle size of approximtely 40 µm) or the microspheres sold under the name Micropearl F 80 ED by Matsumoto; powders formed of natural organic materials, such as powders formed of starch, in particular of crosslinked or non-crosslinked maize, wheat or rice starches, such as the powders formed of starch crosslinked with octenylsuccinic anhydride sold under the name Dry-Flo by National Starch; silicone resin microbeads, such as those sold under the name Tospearl by Toshiba Silicone, in particular Tospearl 240; amino acid powders, such as the lauroyllysine powder sold under the name Amihope LL-11 by Ajinomoto; wax microdispersion particles which preferably have mean dimensions of less than 1 µm and in particular ranging from 0.02 µm to 1 µm and which are essentially composed of a wax or of a mixture of waxes, such as the products sold under the name Aquacer by Byk Cera, in particular Aquacer 520 (mixture of synthetic and natural waxes), Aquacer 514 or 513 (polyethylene wax) or Aquacer 511 (polymer wax), or such as the products sold under the name Jonwax 120 by Johnson Polymer (mixture of polyethylene and paraffin waxes) and under the name Ceraflour 961 by Byk Cera (micronized modified polyethylene wax); and their mixtures.

### Additives

The cosmetic compositions according to the invention can additionally comprise cosmetic adjuvants chosen from softening agents, antioxidants, opacifying agents, stabilizing agents, moisturizing agents, vitamins, bactericides, preservatives, polymers, fragrances, thickening or suspending agents, propellants or any other ingredient normally used in cosmetics for this type of application.

Of course, a person skilled in the art will take care to choose this or these optional additional compounds so that the advantageous properties intrinsically attached to the cosmetic composition in accordance with the invention are not, or not substantially, detrimentally affected by the envisaged addition or additions.

### THICKENERS AND SUSPENDING AGENTS

The thickeners can be chosen from carboxyvinyl polymers, such as the Carbopols (Carbomers) and the Pemulens (acrylate/C₁₀-C₃₀ alkyl acrylate copolymer); polyacrylamides, such as, for example, the crosslinked copolymers sold under the names Sepigel 305 (CTFA name: polyacrylamide/C13-14 isoparaffin/laureth 7) or Simulgel 600 (CTFA name: acrylamide/sodium acryloyldimethyltaurate copolymer/isohexadecane/polysorbate 80) by Seppic; optionally crosslinked and/or neutralized polymers and copolymers of 2-acrylamido-2-methylpropanesulfonic acid, such as the poly(2-acrylamido-2-methylpropanesulfonic acid) sold by Hoechst under the trade name Hostacerin AMPS (CTFA name: ammonium polyacryloyldimethyltaurate) or Simulgel 800, sold by Seppic (CTFA name: sodium polyacryloyldimethyltaurate/polysorbate 80/sorbitan oleate); copolymers of 2-acrylamido-2-methylpropanesulfonic acid and of hydroxyethyl acrylate, such as Simulgel NS and Sepinov EMT 10, sold by Seppic; cellulose derivatives, such as hydroxyethylcellulose or cetyl hydroxyethylcellulose; polysaccharides and in particular gums, such as xanthan gum or hydroxypropyl guar gums; or silicas, such as, for example, Bentone Gel MIO, sold by NL Industries, or Veegum Ultra, sold by Polyplastic.

The thickeners can also be cationic, such as, for example, Polyquaternium-37, sold under the name Salcare SC95 (Polyquaternium-37 (and) Mineral Oil (and) PPG-1 Trideceth-6) or Salcare SC96 (Polyquaternium-37 (and) Propylene Glycol Dicaprylate/Dicaprate (and) PPG-1 Trideceth-6), or other crosslinked cationic polymers, such as, for example, those having the CTFA name Ethyl Acrylate/Dimethylaminoethyl Methacrylate Cationic Copolymer In Emulsion.

### SUSPENSION AGENTS

In order to improve the homogeneity of the product, use may additionally be made of one or more suspending agents which are preferably chosen from hydrophobic modified montmorillonite clays, such as hydrophobic modified bentonites or hectorites. Mention may be made, for example, of the product Stearalkonium Bentonite (CTFA name) (reaction product of bentonite and of the quaternary ammonium stearalkonium chloride), such as the commercial product sold under the name Tixogel MP 250 by Sud Chemie Rheologicals, United Catalysts Inc., or the product Disteardimonium Hectorite (CTFA name) (reaction product of hectorite and of distearyldimonium chloride), sold under the name Bentone 38 or Bentone Gel by Elementis Specialities.

Other suspending agents can be used, in this case in hydrophilic (aqueous and/or ethanolic) media. They can be cellulose, xanthan, guar, starch, locust bean or agar derivatives.

The suspending agents are preferably present in amounts ranging from 0.1% to 5% by weight and more preferentially from 0.2% to 2% by weight, with respect to the total weight of the composition.

The amounts of these various constituents which can be present in the cosmetic composition according to the invention are those conventionally used in compositions for the treatment of perspiration.

### Aerosols

The compositions according to the invention can also be pressurized and be packaged in an aerosol device composed of:
(A) a container comprising a composition as defined above,
(B) at least one propellant and one means for dispensing the said aerosol composition.

The propellants generally used in products of this type, which are well known to a person skilled in the art, are, for example, dimethyl ether (DME); volatile hydrocarbons, such as n-butane, propane or isobutane, and their mixtures, optionally with at least one chlorinated and/or fluorinated hydrocarbon; mention may be made, among the latter, of the compounds sold by DuPont de Nemours under the Freon® and Dymel® names, in particular monofluorotrichloromethane, difluorodichloromethane, tetrafluorodichloroethane and 1,1-difluoroethane, sold in particular under the trade name Dymel 152 A by DuPont. Use may also be made, as propellant, of carbon dioxide gas, nitrous oxide, nitrogen or compressed air.

The compositions comprising perlite particles as defined above and the propellant(s) can be in the same compartment or in different compartments in the aerosol container. According to the invention, the concentration of propellant generally varies from 5% to 95% by weight pressurized and more preferentially from 50% to 85% by weight, with respect to the total weight of the pressurized composition.

The dispensing means, which forms a part of the aerosol device, is generally composed of a dispensing valve controlled by a dispensing head, itself comprising a nozzle via which the aerosol composition is vaporized. The container comprising the pressurized composition can be opaque or transparent. It can be made of glass, of polymer or of metal, optionally covered with a protective lacquer layer.

The expressions "between ... and ..." and "ranging from ... to ..." should be understood as meaning limits included, unless otherwise specified.

The examples which follow illustrate the present invention without limiting the scope thereof.

### I/ IN VITRO SENSORY TESTS ON DEODORANT ACTIVITY OF PETROSELINIC ACID

### a) Strains used in the tests

The four strains of microorganisms which were tested and which are representative of the microorganisms responsible for unpleasant body odours are shown in the following Table 1:

**Table 1:**

| **Species** | **Strain name** | **Reference** |
|---|---|---|
| *Corynebacterium striatum* | LMG 19648 | Voisin *et al.,* 1999 [5] |
| *Corynebacterium mucifaciens* | LMG 19067 | Taylor *et al.,* 2003 [6] |
| *Corynebacterium jeikeium* | LMG 19049 | Natsch *et al.,* 2006 [7] |
| *Staphylococcus epidermidis* | DSMZ 20044 | Verhulst *et al.,* 2009 [8] |

### b) Culturing of the microorganisms

The four microorganisms were each cultured in a liquid medium comprising 1.6 g/I of KH₂PO₄, 5.0 g/l of (NH₄)₂HPO₄, 0.38 g/l of Na₂SO₄, 3.35 g/I of yeast nitrogen base (difco), 0.5 g/I of yeast extract and 0.5 g/l of MgCl₂.6H₂O, 1.0 g/l of Tween 80, 1.0 g/l of valine, 1.0 g/l of leucine, 1.0 g/l of isoleucine and 1.0 g/l of methionine. Depending on the analyses to be carried out (profile of the odour by GC/MS or sensory evaluation), the various types of culture medium were prepared either in 2 ml of culture (high-throughput screening format in 96-well microtitration plates) or else 200 ml in Erlenmeyer flasks. For the GC/MS analyses, 2 ml of culture are sufficient but, for the sensory evaluation, for a panel of 12 people, the minimum amount of 200 ml of culture is necessary.

The petroselinic acid was added to the culture medium before the inoculation of the culture in a concentration known not to inhibit or remove the bacteria.

Other known saturated or polyunsaturated acids were also added to each culture medium in a concentration known not to inhibit or remove the bacteria.

The list of the vitamins which were added to the different *C. striatum. striatum, C. mucifaciens, C. jeikeium* and *S. epidermidis* culture media is given in the following Table 2.

**Table 2**

| **Compound** | **Concentration (g/l)** |
|---|---|
| Linolenic acid | 0.5 |
| Myristic acid | 0.005 |
| Azelaic acid | 0.5 |
| Petroselinic acid | 0.05 |

The strains were cultured aerobically at 37°C for 48 hours. The magnitude of the growth was measured with the optical density (OD) at 600 nm. The cultures were subsequently analyzed for volatile odorous compounds by SPME-GC/MS. The sensory analysis of 200 ml of each culture medium was also carried out.

### c) Analysis by GC/MS of the volatile compounds by Solid Phase MicroExtraction (SPME)

In order to determine the relative amount of volatile compounds present in the culture medium, a solid phase microextraction (SPME; Supelco, USA), gas chromatography coupled with mass spectrometry (HS-SPME-GC/MS; Fisons, USA), is carried out. The solid-phase extraction was carried out with a grey SPME fibre (Carboxen/PDMS/divinylbenzene; Supelco, USA) at 60°C for 15 minutes. Subsequently, the fibre is desorbed at 250°C. The compounds extracted were separated on a 60 mm x 0.32 µm column with a VF-1ms (df = 1 µm) stationary phase (Varian, United States). The GC separation began at 40°C for 2 minutes and then the temperature is increased by 10°C/min up to 250°C. The mass spectra were recorded over an m/z range of 30-250.

For each compound identified, the surface area of the peak was integrated and standardized with respect to internal standards added (D₆-dimethyl sulfide, D₁₂-hexanal, D₅-hexanone, D₆-benzaldehyde or 1,2,3-trichloropropane). This semi-quantitative method is based on data which are expressed in the relative surface area of the peak. Only the samples which were analyzed within a short period of time, one after the other (for example, in one or two days), could be quantitatively compared with one another. The GC/MS method applied made it possible to identify numerous compounds comprising alcohols, aldehydes, sulfur compounds and ketones.

The volatile odour profiles were visualized using heat charts, identifying, on the one hand, the odours produced at high intensity levels and, on the other hand, the odours produced at low intensity levels. In addition, the hierarchical grouping was applied to the odour profiles in order to visualise changes in odour profiles during the addition of specific compounds to the culture medium.

### d) Sensory evaluation of the fermentation samples

For the sensory analysis, the minimum amount of 200 ml of culture medium is necessary for a panel of 12 experts. The jury of experts was selected by an ISO 8586 procedure. Before the sensory analysis, the cultures were sterilized by filtration and the filtrates were stored at 4°C until analyzed. In a first phase, the profile of the odour was evaluated on the basis of the strongest odours by the sensory panel, with an intensity grade which varies from 1 to 4 (1 - a little, 2 - moderate, 3 - strong and 4 - very strong). In addition, each member of the jury of experts indicated the hedonic value of the sample (general attractiveness) from the most unpleasant (grade 1) to the pleasantest (grade 5).

### e) Impact of the addition of the active agents on the production of the odour

### 1. C. ieikeium

The cultures produced by *C. jeikeium* were felt to be the most aggressive with regard to the odour. In contrast to linolenic, myristic and azelaic acids, the addition of petroselinic acid made it possible to significantly improve the attractiveness of the perception of the odour produced by this microorganism.

### 2. C. striatum

The cultures of *C. striatum* produced large amounts of aldehydes under the conditions of the reference media and produce an unpleasant odour of "cheese" and of "sweat". In contrast to linolenic, myristic and azelaic acids, the addition of petroselinic acid made possible a significant reduction in the amount of aldehydes and at a greater concentration of ketone compounds (3-pentan-2-one, 2-undecanone, 2-butanone, 6-methyl-5-hepten-2-one). The resulting odour was perceived as being more pleasant by the panel of experts, with fresher notes with scents described as "green" and "putty".

### 3. S. epidermidis

The cultures of *S. epidermidis* produced smaller amounts of volatile compounds, in comparison with the other three strains, but the description of the odour is similar to that produced by *C. striatum.* In contrast to linolenic, myristic and azelaic acids, the addition of petroselinic acid made possible a significant reduction in the amount of aldehydes (methional, 2-butanal, 2-pentenal, 2-hexenal, 2-methylbutanal and dodecanal) and at a greater concentration of ketone compounds (acetone, 2-butanone, 3-penten-2-one, 2-pentanone, 6-methyl-5-hepten-2-one). The resulting odour was perceived as being more pleasant by the panel of experts, with fresher notes with scents described as "green" and "putty".

### 4. C. mucifaciens

The cultures of *C. mucifaciens* produced small amounts of volatile compounds but the description of the odour is similar to that produced by *C. striatum.* In contrast to linoleic, linolenic, myristic and azelaic acids, the addition of petroselinic acid made it possible to obtain an odour profile with a noticeable reduction in aldehydes and a greater concentration of ketone compounds and a more pleasant perception of the odour with a scent described as "putty".

### I/ IN VIVO SENSORY TESTS ON DEODORANT ACTIVITY OF PETROSELINIC ACID

A sensory evaluation was carried out on the deodorant effect on the scalp of petroselinic acid applied orally by a sniff test on a panel of 44 people.

### Composition tested

Coriander oil assaying 98% of petroselenic acid
Amount topically: 400 mg
Administration twice daily for a period of 28 days

### Protocol:

The panel was formed of 44 volunteers (divided between women and men of all ages), who give off strong odours from the scalp, for which subjects the grading of the item "intensity of the odour" is evaluated at a minimum with the score of 6 24H after the final shampooing, according to the opinion of 5 experts, on a scale ranging from 1 (imperceptible intensity of the odour) to 9 (extremely strong intensity of the odour). Number of groups: 2 groups of 22 subjects (one group which orally ingests coriander oil assayed at 98% petroselinic acid and a control group which ingests a reference placebo not comprising the said coriander oil).

On the one hand, the sensory evaluation of the deodorant effect is carried out by the sniff test on the scalp (5 trained panellists).

Determination of:
- Intensity of the unpleasant odour (scale structured from 1 to 9),
- Description of the malodorous note,
- Quantification of the fragrant note (scale structured from 1 to 9),
- Description of the fragrant note good odour,
- Overall hedonic value (scale structured from 1 to 9).

On the other hand, the volunteers carry out a self-evaluation of the deodorant effect and of the modification to the body odour on the scalp.

Determination of:
- Intensity of the unpleasant odour (scale structured from 1 to 5),
- Description of the malodorous note,
- Quantification of the fragrant note (scale structured from 1 to 5),
- Description of the fragrant note good odour,
- Overall hedonic value (scale structured from 1 to 5).

The results of the tests have shown that coriander oil comprising 98% of petroselinic acid according to the invention makes it possible to effectively reduce the intensity of the odour on the scalp and also the annoyance related to this odour. Thus, the coriander oil comprising 98% of petroselinic acid tested makes it possible to reduce the unpleasant odour on the scalps of the volunteers and to improve the fragrant note.

### II/ COMPARATIVE TESTS ON ANTIMICROBIAL ACTIVITY

The Minimum Inhibitory Concentration (MIC) on the microorganisms *Corynebacterium striatum, Corynebacterium mucifaciens* and *Corynebacterium xerosis* with the following active agents was compared.

The three strains of microorganisms which were tested and which are representative of the microorganisms responsible for unpleasant body odours are shown in the following Table 3:

**Table 3**

| **Species** | **Name of the strain** | **Reference** |
|---|---|---|
| *Corynebacterium striatum* | LMG 19648 | Voisin *et al.,* 1999 [1] |
| *Corynebacterium mucifaciens* | LMG 19067 | Taylor *et al.,* 2003 [2] |
| *Corynebacterium xerosis* | DSM 20743 | Natsch *et al.,* 2006 [3] |

The results are shown in the following Table 4:

**Table 4**

| **Active agent used** | ***C. striatum*** | ***C. mucifaciens*** | ***C. xerosis*** |
|---|---|---|---|
| Coriander essential oil* (outside the invention) commercial reference to be specified | 25 microg/ml | 25 microg/ml | 10 microg/ml |
| Coriander seed oil comprising 79% by weight of petroselinic acid (invention) commercial reference to be specified | non-detectable | non-detectable | non-detectable |
| Petroselinic acid (invention) | non-detectable | non-detectable | non-detectable |
| Triclosan (outside the invention) | 10 microg/ml | 25 microg/ml | 50 microg/ml |

| | | | |
|---|---|---|---|
| *The coriander essential oil used is obtained by steam distillation and has the following composition | | | |

### CHEMICAL ANALYTICAL DATA (%) :

| | | | |
|---|---|---|---|
| α-pinene | 3 to 7 | camphor | 4 to 6 |
| myrcene | 0.5 to 1.5 | α-terpineol | 0.5 to 1.5 |
| limonene | 2 to 5 | geraniol | 0.5 to 3 |
| γ-terpinene | 2 to 7 | geranyl acetate | 1 to 3.5 |
| linalol | 65 to 78 | | |

It has been found that the vegetable oil enriched in petroselinic acid and the petroselinic acid did not have an antimicrobial activity with regard to the strains tested.

### Examples of topical compositions

### Example 1: sticks for the armpits

| **Ingredients** | **Amount (%)** |
|---|---|
| | |
| Coriander seed oil | 10.00 |
| Antioxidant | 0.05 |
| Isopropanol | 40.00 |
| Preservative | 0.30 |
| Water | q.s. for 100 |

### Example 2: aerosol for the armpits

| **Ingredients** | **Amount (%)** |
|---|---|
| Petroselinic acid | 5.00 |
| Glyceryl stearate | 1.00 |
| Oil formed of cetearyl alcohol/oxyethylenated cetearyl alcohol comprising 30 mol of EO (Sinnowax AO®, sold by Henkel) | 3.00 |
| Cetyl alcohol | 1.00 |
| Dimethicone (DC 200 Fluid®, sold by Dow Corning) | 1.00 |
| Isopropyl myristate (Estol IMP 1514®, sold by Uniqema) | 3.00 |
| Antioxidant | 0.05 |
| Preservative | 0.30 |
| Water | q.s. for 100 |

### Example 3: capsule

| **Active principle** | **Amount (%)** |
|---|---|
| Coriander seed oil | 400 mg |

| **Excipient** | |
|---|---|
| Xanthan gum | 0.8 mg |
| Sodium benzoate | 0.2 mg |
| Maltodextrin | q.s. for 30 g |

One capsule can be taken per day.

### Example 4: capsule

| **Ingredients** | **mg/capsule** |
|---|---|
| *Lactobacillus johnsonii* | 108 cfu |
| Coriander seed oil | 300 mg |
| Vitamin C | 60 |
| Magnesium stearate | 0.02 |

One or two of these capsules can be taken per day.

### Example 5: formulation of sugar-coated tablet type

| **Active principles** | **mg/sugar-coated** |
|---|---|
| *Lactobacillus paracaseï* ST11 | 5.108 cfu |
| Caraway oil | 200 |

| **Excipient for the core of the sugar-coated tablet** | |
|---|---|
| Microcrystalline cellulose | 70 |
| Encompress™ | 60 |
| Magnesium stearate | 3 |
| Anhydrous colloidal silica | 1 |

| **Coating agent** | |
|---|---|
| Shellac | 5 |
| Talc | 61 |
| Sucrose | 250 |
| Polyvidone | 6 |
| Titanium dioxide | 0.3 |
| Colouring agent | 5 |

This type of sugar-coated tablet can be taken 1 to 3 times per day.

## Claims

1. Cosmetic use, as deodorant active agent, of a monounsaturated fatty acid or one of its salts and/or one of its esters and/or one of its amides, in which the said monounsaturated fatty acid is chosen from oleic acid, petroselinic acid, sapienic acid, *cis*-8-octadecenoic acid or *cis*-vaccenic acid, or their mixtures.

2. Use according to Claim 1, in which the said monounsaturated fatty acid, its salt and/or its ester and/or its amide is used in an isolated form or in an oil extracted from a plant, said oil comprising at least 40% by weight of the said monounsaturated fatty acid, of its salt and/or of its ester and/or its amide.

3. Use according to either one of the preceding claims, in which the said monounsaturated fatty acid, its salt and/or its ester and/or its amide is used in an oil comprising at least 40% by weight of the said monounsaturated fatty acid, and preferably from 40 to 95% by weight, and more preferably from 65 to 95% by weight of the said monounsaturated fatty acid, of its salt and/or of its ester and/or its amide.

4. Use according to any one of the preceding claims, in which the said monounsaturated fatty acid is petroselinic acid in isolated form or in an oil comprising at least 40% by weight of petroselinic acid, and preferably from 40 to 95% by weight, and more preferably from 65 to 95% by weight of petroselinic acid.

5. Use according to Claim 4, in which the said petroselinic acid is used in the form of an oil of an umbellifer or of *Geranium sanguineum.*

6. Use according to Claim 5, in which the said umbellifer oil is chosen from the seed oils of coriander, chervil, carrot, celery, cumin, caraway, parsley and dill, and their mixtures.

7. Use according to any one of Claims 1 to 6, **characterized in that** the said monounsaturated fatty acid, its salt and/or its ester is present in a composition comprising a physiologically acceptable medium.

8. Use according to Claim 7, where the composition is applied orally.

9. Use according to Claim 8, where the content of the said monounsaturated fatty acid is such that the daily dose varies from 0.5 to 2500 mg/d, in particular from 1 to 2000 mg/d, more particularly from 2 to 1500 mg/d, indeed even from 3 to 1000 mg/d and in particular from 5 to 600 mg/d.

10. Use according to any one of Claims 1 to 7, where the composition is applied topically to the surface of a human keratinous substance.

11. Cosmetic method by the oral route for treating and/or preventing unpleasant human body odours, consisting in ingesting an oral composition comprising, in a physiologically acceptable medium, at least one monounsaturated fatty acid or one of its salts and/or of its esters and/or one of its amides as defined according to any one of the preceding claims.

12. Method according to Claim 11, where the content of monounsaturated fatty acid is such that the daily dose varies from 0.5 to 2500 mg/d, in particular from 1 to 2000 mg/d, more particularly from 2 to 1500 mg/d, indeed even from 3 to 1000 mg/d and in particular from 5 to 600 mg/d.

## Patentansprüche

1. Kosmetische Verwendung einer einfach ungesättigten Fettsäure oder eines ihrer Salze und/oder eines ihrer Ester und/oder eines ihrer Amide als Deodorantwirkstoff, wobei die einfach ungesättigte Fettsäure aus Ölsäure, Petroselinsäure, Sapiensäure, *cis*-8-Octadecensäure oder *cis*-Vaccensäure oder ihren Mischungen ausgewählt ist.

2. Verwendung nach Anspruch 1, wobei die einfach ungesättigte Fettsäure, ihr Salz und/oder ihr Ester und/oder ihr Amid in isolierter Form oder in einem aus einer Pflanze extrahierten Öl verwendet wird, wobei das Öl mindestens 40 Gew.-% der einfach ungesättigten Fettsäure, ihres Salzes und/oder ihres Esters und/oder ihres Amids umfasst.

3. Verwendung nach einem der vorhergehenden Ansprüche, wobei die einfach ungesättigte Fettsäure, ihr Salz und/oder ihr Ester und/oder ihr Amid in isolierter Form oder in einem Öl, das mindestens 40 Gew.-% der einfach ungesättigten Fettsäure und vorzugsweise 40 bis 95 Gew.-% und weiter bevorzugt 65 bis 95 Gew.-% der einfach ungesättigten Fettsäure, ihres Salzes und/oder ihres Esters und/oder ihres Amids umfasst, verwendet wird.

4. Verwendung nach einem der vorhergehenden Ansprüche, wobei es sich bei der einfach ungesättigten Fettsäure um Petroselinsäure in isolierter Form oder in einem Öl, das mindestens 40 Gew.-% Petroselinsäure und vorzugsweise 40 bis 95 Gew.-% und weiter bevorzugt 65 bis 95 Gew.-% Petroselinsäure umfasst, handelt.

5. Verwendung nach Anspruch 4, wobei die Petroselinsäure in Form eines Öls eines Doldenblütlers oder von *Geranium sanguineum* verwendet wird.

6. Verwendung nach Anspruch 5, bei dem das Doldenblütleröl aus den Samenölen von Koriander, Kerbel, Karotte, Sellerie, Kreuzkümmel, Kümmel, Petersilie und Dill und ihren Mischungen ausgewählt ist.

7. Verwendung nach einem der Ansprüche 1 bis 6, **dadurch gekennzeichnet, dass** die einfach ungesättigte Fettsäure, ihr Salz und/oder ihr Ester in einer Zusammensetzung vorliegt, die ein physiologisch unbedenkliches Medium umfasst.

8. Verwendung nach Anspruch 7, wobei die Zusammensetzung oral appliziert wird.

9. Verwendung nach Anspruch 8, wobei der Gehalt der einfach ungesättigten Fettsäure so beschaffen ist, dass die Tagesdosis von 0,5 bis 2500 mg/d, insbesondere von 1 bis 2000 mg/d, spezieller von 2 bis 1500 mg/d, sogar von 3 bis 1000 mg/d und insbesondere von 5 bis 600 mg/d variiert.

10. Verwendung nach einem der Ansprüche 1 bis 7, wobei die Zusammensetzung topisch auf die Oberfläche einer menschlichen Keratinsubstanz appliziert wird.

11. Kosmetisches Verfahren auf oralem Wege zur Behandlung und/oder Prävention von unangenehmen menschlichen Körpergerüchen, das darin besteht, dass man eine orale Zusammensetzung einnimmt, die in einem physiologisch unbedenklichen Medium mindestens eine einfach ungesättigte Fettsäure oder eines ihrer Salze und/oder ihrer Ester und/oder ihrer Amide gemäß einem der vorhergehenden Ansprüche umfasst.

12. Verfahren nach Anspruch 11, wobei der Gehalt der einfach ungesättigten Fettsäure so beschaffen ist, dass die Tagesdosis von 0,5 bis 2500 mg/d, insbesondere von 1 bis 2000 mg/d, spezieller von 2 bis 1500 mg/d, sogar von 3 bis 1000 mg/d und insbesondere von 5 bis 600 mg/d variiert.

## Revendications

1. Utilisation cosmétique, comme agent actif déodorant, d'un acide gras monoinsaturé ou de l'un de ses sels et/ou l'un de ses esters et/ou l'un de ses amides, dans laquelle ledit acide gras monoinsaturé est choisi parmi l'acide oléique, l'acide pétrosélinique, l'acide sapiénique, l'acide *cis*-8-octadécénoique ou l'acide *cis-*vaccénique, ou leurs mélanges.

2. Utilisation selon la revendication 1, dans laquelle ledit acide gras monoinsaturé, son sel et/ou son ester et/ou son amide, est utilisé sous forme isolée ou dans une huile extraite à partir d'une plante, ladite huile comprenant au moins 40% en poids dudit acide gras monoinsaturé, de son sel et/ou son ester et/ou son amide.

3. Utilisation selon l'une ou l'autre des revendications précédentes, dans laquelle ledit acide gras monoinsaturé, son sel et/ou son ester et/ou son amide, est utilisé dans une huile comprenant au moins 40% en poids dudit acide gras monoinsaturé, et préférablement de 40 à 95% en poids, et plus préférablement de 65 à 95% en poids dudit acide gras monoinsaturé, de son sel et/ou de son ester et/ou de son amide.

4. Utilisation selon l'une quelconque des revendications précédentes, dans laquelle ledit acide gras monoinsaturé est l'acide pétrosélinique sous forme isolée ou dans une huile comprenant au moins 40% en poids d'acide pétrosélinique, et préférablement de 40 à 95% en poids, et plus préférablement de 65 à 95% en poids d'acide pétrosélinique.

5. Utilisation selon la revendication 4, dans laquelle ledit acide pétrosélinique est utilisé sous la forme d'une huile d'une ombellifère ou de *Géranium sanguineum.*

6. Utilisation selon la revendication 5, dans laquelle ladite huile d'ombellifère est choisie parmi les huiles de graines de coriandre, de cerfeuil, de carotte, de céleri, de cumin, de carvi, de persil et d'aneth, et leurs mélanges.

7. Utilisation selon l'une quelconque des revendications 1 à 6, **caractérisée en ce que** ledit acide gras monoinsaturé, son sel et/ou son ester est présent dans une composition comprenant un milieu physiologiquement acceptable.

8. Utilisation selon la revendication 7, dans laquelle la composition est appliquée par voie orale.

9. Utilisation selon la revendication 8, dans laquelle la teneur en ledit acide gras monoinsaturé est telle que la dose journalière varie de 0,5 à 2500 mg/jour, en particulier de 1 à 2000 mg/jour, plus particulièrement de 2 à 1500 mg/jour, mieux encore de 3 à 1000 mg/jour et en particulier de 5 à 600 mg/jour.

10. Utilisation selon l'une quelconque des revendications 1 à 7, dans laquelle la composition est appliquée par voie topique à la surface d'une substance kératinique humaine.

11. Méthode cosmétique via voie orale, destinée au traitement et/ou à la prévention d'odeurs corporelles humaine désagréables, consistant en l'ingestion d'une composition orale comprenant, dans un milieu physiologiquement acceptable, au moins un acide gras monoinsaturé ou l'un de ses sels et/ou de ses esters et/ou l'un de ses amides, tels que définis selon l'une quelconque des revendications précédentes.

12. Méthode selon la revendication 11, dans laquelle la teneur en acide gras monoinsaturé est telle que la dose journalière varie de 0,5 à 2500 mg/jour, en particulier de 1 à 2000 mg/jour, plus particulièrement de 2 à 1500 mg/jour, mieux encore de 3 à 1000 mg/jour et en particulier de 5 à 600 mg/jour.
